# EUROPEAN PATENT SPECIFICATION

(11) **EP 2 963 123 B1**
(45) Date of publication and mention of the grant of the patent: **08.04.2020**
(21) Application number: 14756887.7
(22) Date of filing: 28.02.2014
(51) Int. Cl.: C12Q 1/28, C12Q 1/68, G01N 33/53, G01N 33/531, G01N 33/535

(54) **DAB-CONTAINING SUBSTRATE KIT FOR DYEING USE WHICH IS PRODUCED USING LABELLING ENZYME**
MIT EINEM MARKIERUNGSENZYM HERGESTELLTES, DAB-HALTIGES SUBSTRATKIT ZUM FÄRBEN
KIT DE SUBSTRAT CONTENANT DU DAB POUR UTILISATION COMME COLORANT QUI EST PRODUIT EN UTILISANT UNE ENZYME DE MARQUAGE

(30) Priority: 28.02.2013 JP 2013039672
(43) Date of publication of application: 06.01.2016
(73) Proprietor: Nichirei Biosciences Inc., Chuo-ku Tokyo 104-8402 (JP)
(72) Inventor: KASAMATSU, Toshiyuki, Higashimurayama-shi Tokyo 189-0003 (JP); KITANO, Yuriko, Higashimurayama-shi Tokyo 189-0003 (JP)
(74) Representative: Plasseraud IP
(86) International application number: PCT/JP2014/055004
(87) International publication number: WO 2014/133120

(56) References cited:
- WO-A1-02/23156
- WO-A1-2010/094283
- US-A1- 2012 077 211
- US-A1- 2012 171 668

## Description

The present invention relates to a diaminobenzidine (DAB)-containing substrate kit that is capable of suppressing precipitation due to DAB aggregation in DAB-containing substrate solution, reducing nonspecific staining, such as background staining, and enhancing intensity of specific staining, in chromogenic reaction in immunostaining, such as immunohistochemical staining (IHC) or immunocytochemical staining (ICC), or in *in situ* hybridization (ISH), using aperoxidase-labeled antibody, with the use of a chromogenic liquid containing DAB and its sensitizer imidazole.

In the field of pathology or molecular biology, various reagents are used in chromogenic liquids for IHC, ICC, or ISH, depending on the labelling enzymes for chromogenic reaction. The most common labelling enzyme is peroxidase, which is mostly plant-derived horseradish peroxidase (HRP) (molecular weight 40 to 45 kDa), as disclosed in Non-patent Publication 1. A Hydrogen donor for the enzymatic reaction of HRP for routine use is DAB-containing substrate solution containing diaminobenzidine (DAB), which develops brown color, and hydrogen peroxide.

DAB-containing substrate solution is usually provided in a two- or three-component system (DAB and hydrogen peroxide are usually separated for avoiding DAB reaction), and commercially available in highly-concentrated stock solutions for preparation upon use or dry tablets. Examples of such commercial products are Liquid DAB+ Substrate Chromogen System (Code: K3468) (DAKO), DAB Quanto (Code: TA-XXX-QHDX) (THERMO SCIENTIFIC), Stable DAB (Code: 750118) (LIFE TECHNOLOGIES), and DAB substrate kit (NICHIREI BIOSCIENCES, INC.). Compositions of most of such products are not disclosed.

Non-patent Publication 1 discloses use of imidazole as a sensitizer for DAB staining. Experience has shown that imidazole is effective in accelerating DAB reaction but on the other hand causes DAB aggregation to trigger DAB precipitation in reaction liquid after a certain time period. It has also been shown that the high sensitizing effect brings about nonspecific staining, such as background staining, in the area other than the objective substance in the presence of HRP in the tissue.

Patent Publication 1 teaches that a nonionic surfactant may be contained in DAB-containing substrate solution as desired, and Patent Publications 2 and 3 teach that EDTA may be contained in DAB-containing substrate solution. However, no discussion was made on the problems with imidazole in these publications.

Patent publication 4 discloses a deposition media, which is a buffered aqueous media having a pH of 4 to 9, comprising a conjugate, a peroxide compound and DAB.

Patent publication 5 discloses a solution comprising a dewaxing solvent composition and an aldehyde releasing reagent composition which is used to prepare a sample before a histochemical reaction.
Patent Publication 1: JP-3503890-B2
Patent Publication 2: CA-2417671-A1
Patent Publication 3: DE-3812605-A1
Patent publication 4: WO 2010/094283 A1
Patent publication 5: WO 02/23156 A1

Non-patent Publication 1: Hiroshi Nagura, Yoshiyuki Osamura, Yutaka Tsutsumi, "Watanabe-Nakane Kouso Koutai Hou (immunoenzymatic technique)", 4th Ed., Interdisciplinary Planning (2002)

The present invention is as defined in the claims.

It is an object of the present invention to provide a DAB-containing substrate kit that is capable of enhancing intensity of specific staining and reducing nonspecific staining in chromogenic reaction in immunostaining, such as immunohistochemical or immunocytochemical staining, or in *in situ* hybridization, using a peroxidase-labeled antibody, by the use of DAB-containing substrate solution containing DAB and its sensitizer imidazole.

It is another object of the present invention to provide a DAB-containing substrate kit that is capable of enhancing intensity of specific staining, suppressing precipitation due to DAB aggregation in DAB-containing substrate solution, and reducing nonspecific staining, in chromogenic reaction in immunostaining or *in situ* hybridization using a peroxidase-labeled antibody, by the use of DAB-containing substrate solution containing DAB and its sensitizer imidazole.

It is a further object of the present invention to provide a DAB-containing substrate kit that enhances intensity of specific staining, reduces nonspecific staining, such as background staining, and suppresses precipitation due to DAB aggregation in DAB-containing substrate solution for a prolonged period of time, in chromogenic reaction in immunostaining or *in situ* hybridization using a peroxidase-labeled antibody, by the use of DAB-containing substrate solution containing DAB and its sensitizer imidazole, whereby usability and storage stability of the substrate kit are improved.

The present inventors have made intensive researches for achieving the above objects, to find out that presence of a particular chelating agent and a particular nonionic surfactant may reduce nonspecific staining, e.g., background staining, which may occur when glass slide is used, even when imidazole, which is known as a sensitizer, is contained for enhanced specific staining in a composition of DAB-containing substrate solution commonly used for color development in immunostaining, such as immunoenzymatic technique, or *in situ* hybridization. Further, DAB aggregation in prepared DAB-containing substrate solution may be suppressed, and storage stability is significantly improved by providing substrate solution in a system of two or more components with adjusted composition, i.e., no precipitation occurs for two weeks from the mixing of the component solutions when stored in shade at 4 °C, and stainability comparable to that immediately after preparation of DAB-containing substrate solution may be achieved. Thereby the present invention has been completed.

As used herein, DAB-containing substrate solution means substrate solution obtained by mixing, or mixing and diluting, the components of the kit of the present invention. Also as used herein, the term "staining" sometimes mean staining or coloring.

According to the present invention, there is provided a DAB-containing substrate kit for staining using a peroxidase-labeled antibody, such as IHC, ICC, or ISH, said kit comprising: chromophore-containing solution (A) comprising diaminobenzidine (DAB) as a peroxidase chromophore, and water; and chromogenic reagent (B) comprising: at least one chelating agent which is tetraethylenediaminetetraacetic acid disodium salt dihydrate (EDTA-2Na); a polyoxyethylene (abbreviated as POE hereinbelow) alkyl ether nonionic surfactant which is POE (23) lauryl ether (Brij 35); imidazole; hydrogen peroxide; and water, wherein said kit is in a system of two- or more components with at least said chromophore-containing solution (A) and said chromogenic reagent (B) being separately stored (sometimes referred to as a present kit hereinbelow),
wherein the DAB-containing substrate solution obtained by mixing, or mixing and diluting, the component solutions of the DAB-containing substrate kit comprises
- 0.1 to 10 mg/mL of DAB;
- 0.3 to 3 mM of chelating agent;
- 0.1 to 5 mass% of POE alkyl ether nonionic surfactant;
- 5 to 100 mM of imidazole; and
- 0.01 to 0.05 mass% of hydrogen peroxide.

According to the present invention, there is also provided the kit, wherein said chromogenic reagent (B) is in a two-component system wherein chromogenic solution (B1) comprising said hydrogen peroxide and said water, and substrate-stabilizing solution (B2) comprising said chelating agent, said POE alkyl ether nonionic surfactants, said imidazole, and said water, are separately stored, and wherein said kit is in a three-component system.

According to the present invention, there is provided IHC, ICC, or ISH method, wherein the present kit is used in staining tissue, cells, or part thereof with DAB.

According to the present invention, there is also provided a staining method, characterized in that the present kit is used in staining tissue, cells, or part thereof with DAB.

The present kit contains chromophore-containing solution (A) containing a peroxidase chromophore DAB, and water, and chromogenic reagent (B) containing a particular chelating agent, a particular surfactant, imidazole, hydrogen peroxide, and water, and is in a system of two-or more components wherein at least the chromophore-containing solution (A) and the chromogenic reagent (B) are separately stored. Thus the present kit is capable of enhancing the intensity of specific staining, reducing nonspecific staining, and suppressing precipitation due to DAB aggregation in DAB-containing substrate solution, in IHC, ICC, or ISH using peroxidase. Further, the present kit is capable of suppressing the precipitation for a prolonged period of time to improve usability and storage stability. The present kit having such advantages are particularly useful in staining tissue specimens stuck on glass slides by IHC, staining cellular specimens by ICC, or staining tissues or cellular specimens by ISH.

The present invention will now be explained in detail.

The present kit is a DAB-containing substrate kit provided in a system of two- or more components, for staining, with DAB chromophore, particular substances, such as antigens, mRNA, or DNA, present in normal or tumor tissues or cells of biological objects in pathological diagnosis or molecular biological tests, on the basis of staining in IHC or ICC or coloring in ISH, using peroxidase-labeled antibody.

In the present invention, the tissue specimens to be subjected to IHC, cellular specimens to be subjected to ICC, or tissue or cellular specimens to be subjected to ISH have been fixed for preventing denaturalization and then sectioned for use. For example, specimens may preferably be used in the present invention that have been, for the purpose of long-term storage, fixed usually with formalin or alcohol, embedded in embedding medium containing, e.g., paraffin, sectioned, and stuck on a glass slide. The antigen-antibody reaction prior to staining, or antibody reaction after hybridization using a probe labeled with DIG (digoxigenin), may be performed by a known process, such as direct or indirect method, using a peroxidase-labeled antibody. Here, the peroxidase is preferably HRP.

The present kit may preferably be used in indirect immunoenzymatic reaction using a glass slide which the tissue or cellular specimens are stuck on. For example, a specimen fixed with formalin and embedded in paraffin on a glass slide, is subjected to deparaffinization, rehydration, and optionally antigenicity retrieval, to remove endogenous peroxidase. Then the specimen is reacted with a primary antibody or a DIG-labeled probe, and then reacted with peroxidase-labeled polymer which has been conjugated with secondary antibodies or anti-DIG antibodies. The resulting specimen is reacted with DAB-containing substrate solution prepared by mixing the component solutions of the present kit, for specific staining and visualization of the reacted area using peroxidase as a catalyst. After the specific staining for visualization, nuclear staining with hematoxylin or the like, may be carried out for improving morphology observability.

The DAB-containing substrate solution is prepared by mixing the component solutions of the present kit, or by mixing the component concentrated solutions of the present kit with a particular amount of water, such as distilled water.

Examples of the tissues that may be used in staining by IHC or coloring by ISH using the present kit may include large intestine (normal or neoplastic), small intestine (normal), duodenum (normal), colon (normal), stomach (normal or neoplastic), esophagus (normal), tongue (normal), liver (neoplastic), pancreas (normal), kidney (normal) brain (normal), cerebellum (normal), heart (normal), mammary gland (normal or neoplastic), placenta, prostate gland (normal or neoplastic), lung (normal or neoplastic), thyroid gland (normal), tonsil (normal), lymph node (normal), uterine cervix (normal), malignant melanoma, Hodgkin's lymphoma, thymic hyperplasia case, GIST (gastrointestinal stromal tumor), and mesothelioma. Among these, those containing muscular or connective tissues, such as large intestine (normal or neoplastic), small intestine (normal), duodenum (normal), colon (normal), and stomach (normal or neoplastic), are particularly prone to background staining, so that the present kit is particularly effective in staining such tissues.

Preferred examples of the cells suitable for staining by ICC or coloring by ISH using the present kit may include breast cancer cells, lung cancer cells, and lymphoblasts (normal) .

The present kit includes chromophore-containing solution (A) containing a peroxidase chromophore DAB and water. The DAB contained in solution (A) functions as a hydrogen donor in the enzymatic reaction of hydrogen peroxide and peroxidase. Polymerization of DAB specifically colors the peroxidase-bound part in brown.

In the solution (A), the content of DAB is not particularly limited as long as the eventually obtained DAB-containing substrate solution produces chromogenic reaction in staining by IHC or ICC or coloring by ISH, and is 0.1 to 10 mg/mL in terms of the concentration in DAB-containing substrate solution for use. At less than 0.1 mg/mL, sufficient chromogenic reaction may not be produced, whereas at over 10 mg/mL, DAB in the DAB-containing substrate solution may aggregate and precipitate.

In the solution (A), the water may be, for example, distilled or ultrapure water. The amount of the water is sufficient for dissolving DAB in the solution (A), and may suitably be selected so that the content of each constituent is in an appropriate range in the eventual DAB-containing substrate solution.

The solution (A) may optionally contain, in addition to DAB and water, various organic solvents for improving DAB solubility or stability, at a content suitably selected for the purpose.

The present kit includes chromogenic reagent (B) containing a particular chelating agent, POE alkyl ether nonionic surfactant, imidazole, hydrogen peroxide, and water.

The chromogenic reagent (B) may be divided into, for example, chromogenic solution (B1) containing the hydrogen peroxide and the water, and substrate-stabilizing solution (B2) containing the particular chelating agent, the POE alkyl ether nonionic surfactants, the imidazole, and the water.

The present kit may be in a system of any number of components as long as it is in a system of two or more components wherein DAB and hydrogen peroxide are separately stored. In the light of efficiency, a two-component system composed of the chromophore-containing solution (A) and the chromogenic reagent (B), and a three-component system composed of the chromophore-containing solution (A), the chromogenic solution (B1), and the substrate-stabilizing solution (B2), are preferred.

The particular chelating agent contained in the chromogenic reagent (B) or the substrate-stabilizing solution (B2) is EDTA-2Na. This chelating agent particularly suppresses nonspecific binding of DAB, in particular, background staining of tissue specimens stuck on glass slides. Further, in combination with the particular nonionic surfactant to be discussed later, the chelating agent may effectively reduce nonspecific binding of DAB even when the tissue specimen is muscular or connective tissue.

The chelating agent in the chromogenic reagent (B) or the substrate-stabilizing solution (B2) tends to exhibit quantity-dependent enhancement of its functional effects, and its content may suitably be selected, taking the functional effects into consideration. Specifically, the content of the chelating agent is such that the concentration in the DAB-containing substrate solution for use is 0.3 to 3 mM.

The particular surfactant contained in the chromogenic reagent (B) or the substrate-stabilizing solution (B2) is a POE alkyl ether which is POE (23) lauryl ether (Brij 35) . This nonionic surfactant has excellent effects, particularly in DAB-containing substrate solution containing DAB and hydrogen peroxide, of suppressing polymerization or precipitation of DAB in the solution, and of enhancing the staining intensity of DAB. The desired effects cannot be obtained with other surfactants, such as Triton X-100 (4-(1,1,3,3-tetramethylbutyl)phenyl-polyethylene glycol), NP-40 (4-nonylphenyl-polyethylene glycol), or Tween 20 (polyoxyethylenesorbitan monolaurate), oraprotic polar solvents, such as DMSO (dimethyl sulfoxide).

The POE alkyl ether nonionic surfactant in the chromogenic reagent (B) or the substrate-stabilizing solution (B2) tends to exhibit quantity-dependent enhancement of their functional effects, and their content may suitably be selected, taking the functional effects into consideration. The content of the surfactant is such that the concentration in the DAB-containing substrate solution for use in IHC, ICC, or ISH is 0.1 to 5 mass%.

The imidazole contained in the chromogenic reagent (B) or the substrate-stabilizing solution (B2) functions as a sensitizer for enhancing the staining intensity of DAB. The imidazole, having such functional effect, enhances the staining intensity but, on the other hand, induces DAB aggregation in the presence of hydrogen peroxide to cause precipitation in the DAB-containing substrate solution after a certain time period, and also induces nonspecific staining of substances other than the objective substance, such as background staining, in a sample tissues or cells. In view of this, in the present kit, the imidazole is contained in the chromogenic reagent (B) or the substrate-stabilizing solution (B2), containing a combination of the particular chelating agent and the particular nonionic surfactant.

The imidazole in the chromogenic reagent (B) or the substrate-stabilizing solution (B2) exhibits quantity-dependent enhancement of the DAB staining intensity, but too much imidazole may increase the risk of the problems discussed above. Thus the content of the imidazole is such that the concentration in the DAB-containing substrate solution for use in IHC, ICC, or ISH is 5 to 100 mM. In case a higher imidazole concentration is desired, the concentration of the particular chelating agent is also preferably increased within the above-mentioned range.

The hydrogen peroxide contained in the chromogenic reagent (B) or the chromogenic solution (B1) acts on peroxidase to contribute to the chromogenic reaction of DAB. The content of the hydrogen peroxide may be an excess amount, but too much hydrogen peroxide may decrease the intensity of specific staining. On the other hand, when the hydrogen peroxide content is too small, the chromogenic reaction may not proceed sufficiently, and the stability of prepared DAB-containing substrate solution is poor.

In view of the above, the content of the hydrogen peroxide is such that the content in the DAB-containing substrate solution for use in IHC, ICC, or ISH is 0.01 to 0.05 mass%.

The water contained in the chromogenic reagent (B), the chromogenic solution (B1), and the substrate-stabilizing solution (B2) may be, for example, distilled or ultrapure water. The content of the water may suitably be selected so that the content of each constituent is within a suitable range.

In the present kit, for efficiently achieving the desired effects of the present invention, the pH of the chromogenic reagent (B) or the substrate-stabilizing solution (B2) may preferably be adjusted so that the pH of the resulting DAB-containing substrate solution is usually 5 to 9, particularly 6 to 8.

The pH may be adjusted with a pH adjuster, such as hydrochloric acid, or a buffer, such as phosphoric acid, Tris, MOPS, HEPES, ADA, CHES, or MES. A pH adjuster, such as hydrochloric acid, is particularly preferred in view of improved storage stability of the chromogenic reagent (B) containing hydrogen peroxide, or of resulting DAB-containing substrate solution.

Each of the component solutions of the present kit may optionally contain additional constituents as long as the effects of the present invention are not impaired and for providing additional effects. Examples of such additional constituents may include preservatives and disinfectants.

The present kit may be used either in manual or automated process. In particular, the present kit may preferably and conveniently be used in a process using an automated instrument with temperature control, automated immunohistochemical staining instrument, or automated *in situ* hybridization instrument.

The staining or coloring in IHC, ICC, or ISH using the present kit may be carried out by mixing the component solutions of the kit, or mixing and dissolving the component solutions of the kit in a prescribed amount of water, to prepare DAB-containing substrate solution and, for example, in the case of immunoenzymatic method using a specimen tissue or cells on a glass slide, adding the DAB-containing substrate solution dropwise onto the labelled area usually at room temperature (15 to 30 °C), and reacting for 1 to 20 minutes.

The present invention will now be explained in more detail with reference to Examples, Controls, and Comparative Examples, which do not limit the present invention.

In the following examples, evaluations were made as follows.

### Evaluation Item (1)

Intensity of specific staining: The intensity of specific staining of an obtained sample was observed under an optical microscope.

The evaluation was expressed in multiples of the intensity of specific staining in Control 1 or 2 being "+".

### Evaluation Item (2)

Background staining: The background staining of an obtained sample was observed under an optical microscope.

The evaluation was expressed based on Comparative Example 1 or 8 wherein background staining was observed being indicated as "±", and Control 1 or 8 wherein background staining was not observed being indicated as "-", and background staining more than these was indicated as "+", and background staining about twice as much as these was indicated as "2+".

### Evaluation Item (3)

Color of DAB-containing substrate solution: The color of the mixed solution obtained by mixing the component solutions of the kit prepared in Examples and Comparative Examples to be discussed later was visually observed.

### Evaluation Item (4)

### Precipitation in DAB-containing substrate solution:

Conditions of precipitate in the mixed solution obtained by mixing the component solutions of the kit prepared in Examples and Comparative Examples to be discussed later was visually observed.

### Evaluation Item (5)

Storage stability: The storage period was determined in which the intensity of specific staining and the background staining determined of DAB-containing substrate solution prepared from a kit stored at 37 °C were evaluated as comparable to those determined of DAB-containing substrate solution obtained by mixing the components of a freshly prepared kit.

### Preparation of sample for immunohistochemical staining using peroxidase-labeled antibody

### (A) Deparaffinization and rehydration

Formalin-fixed paraffin-embedded human small intestine tissue section was sectioned at 3 µm with a microtome, stuck to a silane-coated glass slide, and dried at 37 °C for 16 hours. The slide was deparaffinized by three-minute standing in xylene layer three times, and then rehydrated by three-minutes standing in ethanol layer four times.

### (B) Washing

After the final standing in ethanol layer, the slide was washed with phosphate buffer at pH 7.6 for 3 minutes three times.

### (C) Immunohistochemical staining

### Manual Immunohistochemical staining

The glass slide from the washing (B), after being drained, was placed in 3% hydrogen peroxide/methanol solution for 10 minutes for reaction to remove endogenous peroxidase, and then washed with pH 7.6 phosphate buffer for 3 minute three times.

After the obtained glass slide was drained, the tissue specimen was encircled using a PAP pen (DAIDO SANGYO CO., LTD.) to form a bank for the reagent.

After the glass slide was drained, 100 µL of mouse-derived primary antibody, anti-actin (smooth muscle) monoclonal antibody (clone 1A4) (trade name, NICHIREI BIOSCIENCES, INC.), was added dropwise onto the glass slide, and reacted at 25 °C for 60 minutes. After the reaction was completed, the glass slide was washed with pH 7.6 phosphate buffer for 3 minutes three times.

After the washed glass slide was drained, 100 µL of a labeled polymer, Simple Stain MAX PO (MULTI) (trade name, NICHIREI BIOSCIENCES, INC.), which was prepared by binding, to an amino acid polymer, peroxidase and Fab' fragments of anti-mouse Ig and anti-rabbit Ig as secondary antibodies, was added dropwise onto the glass slide, and reacted at 25 °C for 30 minutes. After the reaction was completed, the glass slide was washed with pH 7.6 phosphate buffer for 3 minutes three times.

After the washing, the glass slide was drained, and DAB-containing substrate solution prepared from the kit obtained in each of Examples and Comparative Examples was added, as a chromogenic substrate, dropwise onto the glass slide, and reacted at room temperature for 5 minutes. Then the glass slide was washed in running water for 5 minutes. After being drained, for counterstaining, the glass slide was reacted in Mayer's hematoxylin for 30 seconds for nuclear staining, and washed in running water for 5 minutes.

Then, after being drained, the slide was subjected to dehydration and clearing by passage through ethanol layer three times, three-minute standing in ethanol layer once, passage through xylene layer once, and five-minute standing in xylene layer twice, and then mounted in non-aqueous mounting medium (NICHIREI BIOSCIENCES INC.) to obtain a sample.

### Control 1 : DAB-containing substrate kit in three-component system without imidazole, particular chelating agent, and particular surfactant

An amount of DAB was dissolved in distilled water so that the concentration in the eventually-obtained DAB-containing substrate solution was 15 mg/mL, to prepare chromophore-containing solution (A). Separately, chromogenic solution (B1) was prepared by dissolving 0.6 mass% hydrogen peroxide in distilled water, and substrate-stabilizing solution (B2') of pH 7.6 Tris-HCl was prepared. Using a kit of the obtained chromophore-containing solution (A), chromogenic solution (B1), and substrate-stabilizing solution (B2'), a drop of each component solution (about 40 µL) was mixed in 1 mL of distilled water under stirring to prepare DAB-containing substrate solution.

Using the obtained DAB-containing substrate solution, samples for immunohistochemical staining were prepared according to the process described above. The obtained samples were evaluated for Evaluation Items (1) and (2) . The results are shown in Table 1.

### Comparative Example 1: DAB-containing substrate kit in three-component system without particular surfactant

An amount of DAB was dissolved in distilled water so that the concentration of the eventually-obtained DAB-containing substrate solution was 15 mg/mL, to prepare chromophore-containing solution (A). Separately, chromogenic solution (B1) was prepared by dissolving 0.6 mass% hydrogen peroxide in distilled water, and substrate-stabilizing solution (B2') was prepared by dissolving imidazole and EDTA-2Na in distilled water at concentrations of 0.5 M and 20 mM, respectively, and adjusting the pH to 7.5 with hydrochloric acid. Using a kit of the obtained chromophore-containing solution (A), chromogenic solution (B1), and substrate-stabilizing solution (B2'), a drop of each component solution (about 40 µL) was mixed in 1 mL of distilled water under stirring to prepare DAB-containing substrate solution. In the obtained solution, the content of the hydrogen peroxide was 0.024 mass%, the imidazole concentration was 20 mM, the EDTA-2Na concentration was 0.8 mM, and the pH was 7.5.

Using the obtained DAB-containing substrate solution, samples for immunohistochemical staining were prepared according to the process described above. The obtained samples were evaluated for Evaluation Items (1) to (4). The evaluations for Evaluation Items (3) and (4) were made after the lapse of one hour from the preparation of the DAB-containing substrate solution. The results are shown in Tables 1 and 2.

### Example 1 : DAB-containing substrate kit in three-component system containing EDTA-2Na as chelating agent

Instead of the substrate-stabilizing solution (B2'), substrate-stabilizing solution (B2) was prepared by dissolving imidazole, EDTA-2Na, and POE(23) lauryl ether (Brij 35, SIGMA-ALDRICH) in distilled water at concentrations of 0.5 M, 20 mM, and 1 mass%, respectively, and adjusting the pH to 7.5 with hydrochloric acid. Except for the preparation of the solution (B2), DAB-containing substrate solution was prepared using a resulting kit in the same way as in Comparative Example 1. In the obtained solution, the concentration of the hydrogen peroxide was 0.024 mass%, the imidazole concentration was 20 mM, the EDTA-2Na concentration was 0.8 mM, the content of Brij 35 was 0.04 mass%, and the pH was 7.5. The evaluations were made in the same way as in Comparative Example 1. For Evaluation Items (3) and (4), additional evaluations were made after the lapse of three days from the preparation of the DAB-containing substrate solution. The results are shown in Tables 1 and 2.

### Comparative Examples 2 to 4: DAB-containing substrate kit in three-component system containing different surfactant

Substrate-stabilizing solution (B2') was prepared in the same way as in Example 1, except that Brij 35 was replaced with polyoxysorbitan nonionic surfactant Tween 20, polyoxyalkylphenyl ether nonionic surfactant Triton X-100, or a sucrose fatty acid ester "DK ester SS" (trade name, DKS CO., LTD.), as shown in Table 1.

Chromophore-containing solution (A) and chromogenic solution (B1) were prepared in the same way as in Comparative Example 1, to obtain a kit in a three-component system. DAB-containing substrate solution was prepared using the obtained kit in the same way as in Comparative Example 1, and the evaluations were made in the same way as in Example 1. The results are shown in Tables 1 and 2. The concentrations in the obtained DAB-containing substrate solution were the same as those in Example 1.

### Examples 2 to 5: DAB-containing substrate kit in three-component system with kind and concentration of chelating agent changed from Example 1

Substrate-stabilizing solution (B2) was prepared in the same way as in Example 1, except that 20 mM of EDTA-2Na was replaced with EDTA-2NH₄, NTA, GEDTA, or EDTA-2Na at a concentration shown in Table 1. Chromophore-containing solution (A) and chromogenic solution (B1) were prepared in the same way as in Comparative Example 1, to obtain a kit in a three-component system. DAB-containingsubstrate solution was prepared using the obtained kit in the same way as in Comparative Example 1, and the evaluations were made in the same way as in Example 1. The results are shown in Tables 1 and 2. In the DAB-containing substrate solution prepared in Example 5, the hydrogen peroxide concentration was 0.024 mass%, the imidazole concentration was 20 mM, the EDTA-2Na concentration was 0.8 mM, the Brij 35 content was 0.04 mass%, and the pH was 7.5.

### Comparative Examples 5 to 7: DAB-containing substrate kit in three-component system with different chelating agent

Substrate-stabilizing solution (B2') was prepared in the same way as in Comparative Example 2, except that EDTA-2Na was replaced with citric acid, tartaric acid, or gallic acid at a concentration shown in Table 1. Chromophore-containing solution (A) and chromogenic solution (B1) were prepared in the same way as in Comparative Example 1, to obtain a kit in a three-component system. DAB-containing substrate solution was prepared using the obtained kit in the same way as in Comparative Example 1, and the evaluations were made in the same way as in Example 1, except that the evaluations of the solution color and the precipitate conditions after the lapse of one hour after the mixing were not made. The results are shown in Tables 1 and 2. In the DAB-containing substrate solutions obtained in Comparative Examples 5 to 7, the hydrogen peroxide concentration was 0.024 mass%, the imidazole concentration was 20 mM, the citric or tartaric acid concentration was 0.8 mM, the gallic acid concentration was 0.08 mM, and the content of Tween 20 was 0.04 mass%.

**Table 1**

| | Kind of surfactant in solution (B2) or (B2') | Kind and concentration of chelating agent in solution (B2) or (B2') | Specific staining intensity | Background staining |
|---|---|---|---|---|
| Control 1 | - | - | + | - |
| Comp. Ex. 1 | - | 20 mM EDTA-2Na | 2.5+ | ± |
| Example 1 | Brij 35 | 20 mM EDTA-2Na | 3+ | - |
| Comp. Ex. 2 | Tween 20 | 20 mM EDTA-2Na | 3+ | + |
| Comp. Ex. 3 | Triton X-100 | 20 mM EDTA-2Na | 3+ | + |
| Comp. Ex. 4 | DK ester SS | 20 mM EDTA-2Na | 3+ | + |
| Example 2 | Brij 35 | 20 mM EDTA-2NH₄ | 3+ | - |
| Example 3 | Brij 35 | 20 mM NTA | 3+ | - |
| Example 4 | Brij 35 | 20 mM GEDTA | 3+ | - |
| Example 5 | Brij 35 | 50 mM EDTA-2Na | 3+ | - |
| Comp. Ex. 5 | Tween 20 | 20 mM citric acid | 2+ | + |
| Comp. Ex. 6 | Tween 20 | 20 mM tartaric acid | 2+ | + |
| Comp. Ex. 7 | Tween 20 | 2 mM gallic acid | + | 2+ |

**Table 2**

| | Solution after 1 hour from mixing | | Solution after 3 days from mixing | |
|---|---|---|---|---|
| | Color | Condition of precipitate | Color | Condition of precipitate |
| Comp. Ex. 1 | Transparent solution | Precipitated | - | - |
| Example 1 | Clear and colorless | No precipitate | Clear Brown | No precipitate |
| Comp. Ex. 2 | Transparent solution | No precipitate | Turbid Brown | Precipitated |
| Comp. Ex. 3 | Transparent solution | No precipitate | Turbid Brown | Precipitated |
| Comp. Ex. 4 | Transparent solution | No precipitate | Turbid Brown | Much precipitate |
| Example 2 | Clear and colorless | No precipitate | Clear Brown | No precipitate |
| Example 3 | Clear and colorless | No precipitate | Clear Brown | No precipitate |
| Example 4 | Clear and colorless | No precipitate | Clear Brown | No precipitate |
| Example 5 | Clear and colorless | No precipitate | Clear Brown | No precipitate |
| Comp. Ex. 5 | - | - | Turbid Brown | Precipitated |
| Comp. Ex. 6 | - | - | Turbid Brown | Precipitated |
| Comp. Ex. 7 | - | - | Turbid Brown | Precipitated |

Tables 1 and 2 show that in Control 1 without imidazole having a staining enhancing effect, the intensity of specific staining was low, but background staining was not observed. In contrast, in Comparative Example 1 with imidazole, background staining was observed even though the particular chelating agent EDTA-2Na was contained, and precipitation was observed in the solution in as a short time as one hour after the mixing. Comparative Examples 3 and 4 with the nonionic surfactant other than the POE alkyl ether nonionic surfactants exhibited still more excellent intensity of specific staining compared to Comparative Example 1, but background staining was also enhanced, and precipitation was observed in the solutions after the lapse of three days from the mixing. Comparative Examples 5 and 6 with the chelating agent having less chelating effect compared to EDTA-2Na, exhibited a lower specific staining intensity compared to Comparative Example 2 with EDTA-2Na, but background staining and solution conditions comparable to Comparative Example 2. In contrast, in Example 1, the specific staining intensity was enhanced, background staining was not observed, and precipitation was not observed in the solution after the lapse of three days from the mixing. Further, Examples 2 to 4 demonstrate that the particular chelating agents other than EDTA-2Na also exhibited the effects comparable to Example 1.

### Example 6: DAB-containing substrate kit in three-component system with different imidazole concentration

Substrate-stabilizing solution (B2) was prepared in the same way as in Example 1, except that the imidazole concentration of 0.5 M was changed to 0.1 M (the imidazole concentration in the DAB-containing substrate solution was 4 mM), 0.2 M (the imidazole concentration in the DAB-containing substrate solution was 8 mM), 0.3 M (the imidazole concentration in the DAB-containing substrate solution was 12 mM), or 0.4 M (the imidazole concentration in the DAB-containing substrate solution was 16 mM), and the evaluations were made in the same way as in Example 1 as a kit in a three-component system. The results show that the specific staining intensity was in the range of +1.5 to +2.5, increasing in a quantity-dependent manner as the imidazole concentration increased. The background staining, the color of the mixed solution, and the precipitation conditions were comparable to those in Example 1.

### Examples 7 to 13: DAB-containing substrate kit in three-component system with different surfactant concentration

Substrate-stabilizing solution (B2) was prepared in the same way as in Example 1, except that the concentration of Brij 35 was changed to the concentration shown in Table 3. Chromophore-containing solution (A) and chromogenic solution (B1) were prepared in the same way as in Comparative Example 1, to obtain a kit in a three-component system. DAB-containing substrate solution was prepared using the obtained kit, and samples for immunohistochemical staining were prepared using the solution immediately after the mixing. The samples were evaluated for Evaluation Items (1) and (2). The results are shown in Table 3. Further, samples for immunohistochemical staining were also prepared using DAB-containing substrate solution stored in shade at 4 °C for 2 weeks after the preparation. The obtained samples were evaluated for Evaluation items (1) and (2), and the solution stored in shade at 4 °C for 2 weeks was subjected to Evaluation Items (3) and (4). The results are shown Table 4. In the obtained DAB-containing substrate solution, the Brij 35 concentration was 0.02 mass% in Example 7, 0.06 mass% in Example 8, 0.08 mass% in Example 9, 0.1 mass% in Example 10, 0.12 mass% in Example 11, 0.2 mass% in Example 12, and 0.4 mass% in Example 13.

**Table 3**

| | Brij 35 concentration (mass%) | Specific staining intensity | Background staining |
|---|---|---|---|
| Example 7 | 0.5 | 3+ | - |
| Example 8 | 1.5 | 3+ | - |
| Example 9 | 2.0 | 3+ | - |
| Example 10 | 2.5 | 3+ | - |
| Example 11 | 3.0 | 3.5+ | - |
| Example 12 | 5.0 | 3.5+ | - |
| Example 13 | 10.0 | 3.5+ | - |

**Table 4**

| | Brij 35 concentration (mass%) | After storage in shade at 4 °C for 2weeks after mixing | | |
|---|---|---|---|---|
| | | Specific staining intensity | Background staining | Condition of precipitation after storage of solution |
| Example 7 | 0.5 | 3+ | - | Precipitated |
| Example 8 | 1.5 | 3+ | - | Precipitated |
| Example 9 | 2.0 | 3+ | - | Precipitated |
| Example 10 | 2.5 | 3+ | - | Slight precipitate |
| Example 11 | 3.0 | 3.5+ | - | No precipitate |
| Example 12 | 5.0 | 3.5+ | - | No precipitate |
| Example 13 | 10.0 | 3.5+ | - | No precipitate |

Table 4 shows that with a higher concentration of the particular surfactant, no precipitate was observed in the DAB-containing substrate solution even after a long-term storage, exhibiting good storage stability.

The kit of Example 13 was further subjected to an accelerated test at 37 °C for 3 months and a freeze-thaw test of storage at -20 °C for 3 days, and the component solutions were mixed and evaluated. The results show no problem in the stainability and precipitation. Further, after the accelerated test and the freeze-thaw test, the mixed solution was stored in shade at 4 °C for 5 days, and evaluated, resulting in little decrease in the specific staining intensity and no background staining. However, with mixed solution after the lapse of 7 days, staining was observed with a slight decrease in the specific staining intensity compared to the solution prepared on the day of the evaluation.

### Example 14: DAB-containing substrate kit in two-component system

An amount of DAB was dissolved in distilled water so that the concentration of the eventually-obtained DAB-containing substrate solution was 20 mg/mL, to prepare chromophore-containing solution (A). Chromogenic reagent (B) was prepared by dissolving hydrogen peroxide, imidazole, EDTA-2Na, and Brij 35 in distilled water at concentrations of 0.025 mass%, 20 mM, 2 mM, and 0.4 mass%, respectively, and adjusting the pH to 7.5 solely with hydrochloric acid. Using a kit in a two-component system composed of the obtained chromophore-containing solution (A) and the chromogenic solution (B), 500 µL of the solution (B) was mixed with a drop of the solution (A) (about 20 µL) under stirring to prepare DAB-containing substrate solution. In the obtained solution, the content of the hydrogen peroxide was 0.025 mass%, the imidazole concentration was 20 mM, the EDTA-2Na concentration was 2 mM, the Brij 35 content was 0.4 mass%, and the pH was 7.5.

Using the obtained DAB-containing substrate solution, samples for immunohistochemical staining were prepared according to the process described above. The obtained samples were evaluated for Evaluation Items (1) and (2), and the obtained kit was subjected to the storage stability test of Evaluation Item (5). The results are shown in Table 5.

### Examples 15 to 18

Chromogenic reagent (B) was prepared in the same way as in Example 14, except that, instead of adjusting the pH to 7.5 solely with hydrochloric acid, the buffer solution shown in Table 5 was titrated with HCl or NaOH to have the pH shown in Table 5. The remaining preparation of chromophore-containing solution (A) and evaluations were made in the same way as in Example 14. The results are shown in Table 5.

**Table 5**

| | Buffer (pH) | Specific staining intensity | Background staining | 37 °C Conservable Period Evaluation Item (5) |
|---|---|---|---|---|
| Example 14 | - (7.5) | 3.5+ | - | Conservable for 3 mths |
| Example 15 | MOPS (7.5) | 3.5+ | - | Conservable for about 2 mths |
| Example 16 | HEPES (7.5) | 3.5+ | - | Conservable for about 1 mth |
| Example 17 | ADA (7.5) | 3.5+ | - | Conservable for about 1 mth |
| Example 18 | CHES (9.0) | 3.5+ | - | Conservable for about 2 mths |

| | | | | |
|---|---|---|---|---|
| MOPS: 3-morphorino propanesulfonic acid HEPES: 4-(2-hydroxyethyl)-1-piperazine-ethanesulphonic acid ADA: N-(2-acetamide) imidodiacetate CHES: N-cyclohexyl-2-aminoethanesulphonic acid | | | | |

Table 5 shows that all the chromogenic reagents (B) exhibited excellent storage stability in the storage test, with Example 14 without a buffer being particularly excellent.

### Example 19

Chromogenic reagent (B) was prepared in the same way as in Example 14, except that the EDTA-2Na concentration of 2 mM was changed to 1 mM or 1.5 mM. The chromophore-containing solution (A) was prepared and the evaluations of the specific staining intensity and the background staining were made, to give the results similar to those in Example 14. The color tone of the staining was observed to find out that the staining was in ocher up to the EDTA-2Na concentration of 1 mM, but in dark brown at 1.5 mM or higher, showing more preferable color tone.

### Manual CISH (Chromogenic in situ Hybridization)

### (A) Preparation of tissue section, deparaffinization, and rehydration

Formalin-fixed paraffin-embedded human breast cancer tissue section confirmed as HER2 positive was sectioned at 5 µm with a microtome, stuck on a silane-coated glass slide, and dried at 37 °C for 16 hours. The slide was deparaffinized by three-minute standing in xylene three times, and then rehydrated by three-minutes standing in ethanol four times.

### (B) Pretreatment

After the final standing in ethanol, the slide was drained, placed in 3% hydrogen peroxide/water to remove endogenous peroxidase, and reacted at 25 °C for 5 minutes. Then, the slide was washed with phosphate buffer (pH 7.6) for 1 minute twice.

After being drained, the slide was placed in 10 mM citrate buffer (pH 6.0) previously warmed to 98 °C in a warm bath, and reacted for 30 minutes. Then, the slide was washed in phosphate buffer (pH 7.6) for 2 minute twice.

After the slide was drained, 100 µL of five-fold diluted protease (NICHIREI BIOSCIENCES, INC.) solution was added dropwise onto the slide, and reacted at 25 °C for 3 minutes, and then washed in phosphate buffer (pH 7.6) for five minutes three times.

For dehydration, the slide was reacted with 70 %, 90 %, and 100 % ethanol (the balance being deionized water) at 25 °C for 1 minute each, and subjected to direct cold wind from a drier to dry the tissue.

### Denaturalization and Hybridization

A HER2 probe was prepared by DIG-labeling, with a linker, an about 220 kb sequence complementary to a region on human chromosome 17 (17q21.1), and 10 µL of the HER2 probe thus obtained was added dropwise around the dried tissue.

A 22 mm × 22 mm cover glass (MATSUNAMI GLASS IND., LTD.) was placed over the tissue, excluding air bubbles, and sealed with a paper bond around the periphery. Then the glass slide was placed on a hot plate set at 75 °C, and thermally denatured for 5 minutes. Then, the glass slide was transferred into a moist chamber, and reacted overnight at 37 °C.

### Post-hybridization and Detection

Next, the paper bond was carefully peeled off, and the glass slide was reacted in 2X SSC buffer (citrate buffer) at 25 °C for 5 minutes, then in 2X SSC buffer previously warmed to 72 °C for 5 minutes, and washed with phosphate buffer (pH 7.6) for 1 minute twice.

An amino acid polymer was conjugated with peroxidase and Fab' fragment of anti-DIG antibody to prepare a labeled polymer, and 100 µL of the labeled polymer thus obtained was added dropwise onto the slide, reacted at 25 °C for 30 minutes, and washed with phosphate buffer (pH 7.6) for 1 minute three times.

Before the reaction, DAB-containing substrate solution was prepared from a kit composed of the component solutions prepared in Examples and Comparative Examples. After the glass slide was drained, 100 µL of the DAB-containing substrate solution was added dropwise on the slide, reacted at room temperature for 10 minutes, and then washed in running water for 5 minutes.

After drained, for counterstaining, the glass slide was reacted in Mayer's hematoxylin for 15 seconds for nuclear staining, and washed in running water for 5 minutes.

After the washing, the slide was drained, and subjected to dehydration and clearing by passage through ethanol three times, three-minute standing in ethanol once, passage through xylene once, and five-minute standing in xylene twice. The slide was then mounted in non-aqueous mounting medium (NICHIREI BIOSCIENCES, INC.) to obtain a sample.

### Control 2: DAB-containing substrate kit in three-component system without imidazole, particular chelating agent, and particular surfactant

An amount of DAB was dissolved in distilled water so that the concentration of the eventually-obtained DAB-containing substrate solution was 15 mg/mL, to prepare chromophore-containing solution (A). Separately, chromogenic solution (B1) was prepared by dissolving 0.6 mass% hydrogen peroxide in distilled water, and substrate-stabilizing solution (B2') of pH 7.6 Tris-HCl was prepared. Using a kit of the obtained chromophore-containing solution (A), chromogenic solution (B1), and substrate-stabilizing solution (B2'), a drop of each component solution (about 40 µL) was mixed in 1 mL of distilled water under stirring to prepare DAB-containing substrate solution.

Using the obtained DAB-containing substrate solution, samples for CISH were prepared according to the process described above. The obtained samples were evaluated for Evaluation Items (1) and (2). The results are shown in Table 6.

### Comparative Example 8: DAB-containing substrate kit in three-component system without particular surfactant

An amount of DAB was dissolved in distilled water so that concentration of the eventually-obtained DAB-containing substrate solution was 15 mg/mL, to prepare chromophore-containing solution (A). Separately, chromogenic solution (B1) was prepared by dissolving 0.6 mass% hydrogen peroxide in distilled water, and substrate-stabilizing solution (B2') was prepared by dissolving imidazole and EDTA-2Na in distilled water at concentrations of 0.5 M and 20 mM, respectively, and adjusting the pH to 7.5 with hydrochloric acid. Using a kit of the obtained chromophore-containing solution (A), chromogenic solution (B1), and substrate-stabilizing solution (B2'), a drop of each component solution (about 40 µL) was mixed in 1 mL of distilled water under stirring to prepare DAB-containing substrate solution. In the obtained solution, the content of the hydrogen peroxide was 0.024 mass%, the imidazole concentration was 20 mM, the EDTA-2Na concentration was 0.8 mM, and the pH was 7.5.

Using the obtained DAB-containing substrate solution, samples for CISH were prepared according to the process described above. The obtained samples were evaluated for Evaluation Items (1) to (4). The evaluations for Evaluation Items (3) and (4) were made after the lapse of one hour from the preparation of the DAB-containing substrate solution. The results are shown in Table 6.

### Example 20: DAB-containing substrate kit in three-component system containing EDTA-2Na as chelating agent

Instead of the substrate-stabilizing solution (B2'), substrate-stabilizing solution (B2) was prepared by dissolving imidazole, EDTA-2Na, and Brij 35 (SIGMA-ALDRICH) in distilled water at concentrations of 0.5 M, 20 mM, and 10 mass%, respectively, and adjusting the pH to 7.5 with hydrochloric acid. Except for the preparation of the solution (B2), DAB-containingsubstrate solution was prepared using a resulting kit in the same way as in Comparative Example 8. In the obtained solution, the concentration of the hydrogen peroxide was 0.024 mass%, the imidazole concentration was 20 mM, the EDTA-2Na concentration was 0.8 mM, the content of Brij 35 was 0.4 mass%, and the pH was 7.5. The evaluations were made in the same way as in Comparative Example 8. For Evaluation Items (3) and (4), additional evaluations were made after the lapse of three days from the preparation of the DAB-containing substrate solution. The results are shown in Tables 6 and 7.

**Table 6**

| | Kind of surfactant in solution (B2) or (B2') | Kind and concentration of chelating agent in solution (B2) or (B2') | Specific staining intensity | Background staining |
|---|---|---|---|---|
| Control 2 | - | - | + | - |
| Comp. Ex. 8 | - | 20 mM EDTA-2Na | 2.5+ | ± |
| Example 20 | Brij 35 | 20 mM EDTA-2Na | 3+ | - |

**Table 7**

| | Solution after 1 hour from mixing | | Solution after 3 days from mixing | |
|---|---|---|---|---|
| | Color | Condition of precipitate | Color | Condition of precipitate |
| Comp. Ex. 8 | Transparent solution | Precipitated | - | - |
| Example 20 | Clear and colorless | No precipitate | Clear Brown | No precipitate |

The results show that the kit of the present invention, also when used in CISH, enhanced the intensity of specific staining, reduced background staining, and suppressed precipitation in the DAB-containing substrate solution.

## Claims

1. A diaminobenzidine (DAB) -containing substrate kit for staining using a peroxidase-labeled antibody, said kit comprising:
chromophore-containing solution (A) comprising DAB as a peroxidase chromophore, and water; and
chromogenic reagent (B) comprising: a chelating agent which is ethylenediaminetetraacetic acid disodium salt dihydrate (EDTA-2Na) ; a polyoxyethylene (POE) alkyl ether nonionic surfactant which is POE (23) lauryl ether (Brij 35); imidazole; hydrogen peroxide; and water,
wherein said kit is in a system of two- or more components with at least said chromophore-containing solution (A) and said chromogenic reagent (B) being separately stored,
wherein the DAB-containing substrate solution obtained by mixing, or mixing and diluting, the component solutions of the DAB-containing substrate kit comprises
- 0.1 to 10 mg/mL of DAB;
- 0.3 to 3 mM of chelating agent;
- 0.1 to 5 mass% of POE alkyl ether nonionic surfactant;
- 5 to 100 mM of imidazole; and
- 0.01 to 0.05 mass% of hydrogen peroxide.

2. The kit according to claim 1, wherein said chromogenic reagent (B) is in a two-component system wherein chromogenic solution (B1) comprising said hydrogen peroxide and said water, and substrate-stabilizing solution (B2) comprising said chelating agent, said POE alkyl ether nonionic surfactant, said imidazole, and said water, are separately stored, and wherein said kit is in a three-component system.

3. The kit according to claim 1 or 2, wherein a pH of the chromogenic reagent (B) or the substrate-stabilizing solution (B2) is adjusted such that a pH of DAB-containing substrate solution for use, prepared by mixing or mixing and diluting the component solutions of the DAB-containing substrate kit for staining, is 5 to 9.

4. Use of the kit according to any one of claims 1 to 3 in immunostaining or staining in *in situ* hybridization.

## Patentansprüche

1. Diaminobenzidin (DAB)-enthaltender Substratkit zum Färben unter Verwendung eines Peroxidase-markierten Antikörpers, wobei der Kit umfasst:
Chromophor-enthaltende Lösung (A), die DAB als ein Peroxidase-Chromophor und Wasser enthält; und
chromogenes Reagenz (B), umfassend: einen Chelatbildner, der Ethylendiamintetraessigsäure-dinatriumsalz-dihydrat (EDTA-2Na) ist; ein nichtionisches Polyoxyethylen (POE)-Alkylether-oberflächenaktives Mittel, das POE (23)-Laurylether (Brij 35) ist; Imidazol; Wasserstoffperoxid; und Wasser,
worin der Kit in einem System aus zwei oder mehr Komponenten vorliegt, wobei mindestens die Chromophor-enthaltend Lösung (A) und das chromogene Reagenz (B) getrennt gelagert werden,
worin die DAB-enthaltende Substratlösung, die durch Mischen oder Mischen und Verdünnen der Komponentenlösungen des DAB-enthaltenten Substratkits erhalten wird, umfasst
- 0,1 bis 10 mg/ml DAB
- 0,3 bis 3 mM Chelatbildner;
- 0,1 bis 5 Masse-% nichtionisches POE-Alkylether-oberflächenaktives Mittel
- 5 bis 100 mM Imidazol; und
- 0,01 bis 0,05 Masse-% Wasserstoffperoxid .

2. Kit nach Anspruch 1, worin das chromogene Reagenz (B) in einem Zweikomponentensystem ist, worin die chromogene Lösung (B 1), die das Wasserstoffperoxid und das Wasser umfasst, und die substratstabilisierende Lösung (B 2), die den Chelatbildner, das nichtionische POE-Alkyletheroberflächenaktive Mittel, das Imidazol und das Wasser umfasst, getrennt gelagert sind, und wobei der Kit in einem Dreikomponentensystem vorliegt.

3. Kit nach Anspruch 1 oder 2, worin ein pH-Wert des chromogenes Reagenz (B) oder der substratstabilisierenden Lösung (B2) so eingestellt ist, dass der pH-Wert der zu verwendenden DAB-haltigen Substratlösung, die durch Mischen oder Mischen und Verdünnen der Komponentenlösungen des DABenthaltenden Substratkits für die Färbung hergestellt wird, 5 bis 9 beträgt.

4. Verwendung des Kits nach einem der Ansprüche 1 bis 3 bei der Immunfärbung oder der Färbung bei In-situ-Hybridisierung.

## Revendications

1. Kit de substrat contenant de la diaminobenzidine (DAB) pour coloration par utilisation d'un anticorps conjugué à une peroxydase, ledit kit comprenant :
une solution contenant un chromophore (A) comprenant de la DAB en tant que chromophore pour peroxydase, et de l'eau ; et
un réactif chromogène (B) comprenant : un agent chélatant qui est le sel disodique dihydraté de l'acide éthylènediaminetétraacétique (EDTA-2Na) ; un tensioactif non ionique de type polyoxyéthylène (POE) alkyléther qui est le POE (23) lauryléther (Brij 35) ; de l'imidazole ; du peroxyde d'hydrogène ; et de l'eau,
dans lequel ledit kit est dans un système à deux composants ou plus avec au moins ladite solution contenant un chromophore (A) et ledit réactif chromogène (B) stockés séparément,
dans lequel la solution substrat contenant de la DAB obtenue par le mélange, ou le mélange et la dilution, des solutions de composant du kit de substrat contenant de la DAB comprend
- 0,1 à 10 mg/ml de DAB ;
- 0,3 à 3 mM d'agent chélatant ;
- 0,1 à 5 % en masse d'un tensioactif non ionique de type POE alkyléther ;
- 5 à 100 mM d'imidazole ; et
- 0,01 à 0,05 % en masse de peroxyde d'hydrogène.

2. Kit selon la revendication 1, dans lequel ledit réactif chromogène (B) est dans un système à deux composants dans lequel une solution chromogène (B1) comprenant ledit peroxyde d'hydrogène et ladite eau, et une solution de stabilisation de substrat (B2) comprenant ledit agent chélatant, ledit tensioactif non ionique de type POE alkyléther, ledit imidazole, et ladite eau, sont stockés séparément, et dans lequel ledit kit est dans un système à trois composants.

3. Kit selon la revendication 1 ou 2, dans lequel un pH du réactif chromogène (B) ou de la solution de stabilisation de substrat (B2) est ajusté de telle sorte qu'un pH de la solution substrat contenant de la DAB à utiliser, préparée par le mélange ou le mélange et la dilution des solutions de composant du kit de substrat contenant de la DAB pour coloration, est de 5 à 9.

4. Utilisation du kit selon l'une quelconque des revendications 1 à 3 dans une immunocoloration ou dans une coloration dans une hybridation *in situ.*
